# EUROPEAN PATENT SPECIFICATION

(11) **EP 1 696 940 B1**
(45) Date of publication and mention of the grant of the patent: **21.03.2007**
(21) Application number: 04804944.9
(22) Date of filing: 20.12.2004
(51) Int. Cl.: A61K 35/16, A61P 7/02

(54) **A UNIVERSALLY APPLICABLE VIRUS INACTIVATED BLOOD PLASMA PRODUCED FROM PORTIONS OF NON-CAUCASIAN PLASMA**
UNIVERSELL EINSETZBARES VIRUS-INAKTIVIERTES BLUTPLASMA AUS TEILEN VON PLASMA, DAS NICHT VON WEISSHÄUTIGEN STAMMT
PLASMA SANGUIN UNIVERSELLEMENT APPLICABLE, VIRUS INACTIVE ET PRODUIT DES PORTIONS DE PLASMA DES NON-CAUCASIENS

(30) Priority: 19.12.2003 EP 03029359
(43) Date of publication of application: 06.09.2006
(73) Proprietor: Octapharma AG, 8853 Lachen (CH)
(72) Inventor: HEGER, Andrea, A-1110 Wien (AT); RÖMISCH, Jürgen, 2440 Gramatneusiedl (AT); SVAE, Tor-Einar, A-2340 Mödling (AT); MARGUERRE, Wolfgang, 69120 Heidelberg (DE)
(74) Representative: Meyers, Hans-Wilhelm
(86) International application number: PCT/EP2004/053608
(87) International publication number: WO 2005/058334

(56) References cited:
- WO-A-99/07390
- DATABASE WPI Section Ch, Week 200219 Derwent Publications Ltd., London, GB; Class B04, AN 2002-140576 XP002280416 & CN 1 321 468 A (DU Z) 14 November 2001 (2001-11-14)
- BERKOW R ET AL: "THE MERCK MANUAL OF DIAGNOSIS AND THERAPY, FIFTEENTH EDITION. PASSAGE TEXT" MERCK MANUAL OF DIAGNOSIS AND THERAPY, RAHWAY, MERCK & CO, US, 1987, page 1132, XP002054540
- BURNOUF T ET AL: "L'INACTIVATION DES VIRUS DANS LES FRACTIONS PLASMATIQUES A USAGE THERAPEUTIQUE VIRAL INACTIVATION ON PLASMA FRACTIONS FOR THERAPEUTIC USE" JOURNAL OF EXPERIMENTAL AND CLINICAL HEMATOLOGY / NOUVELLE REVUE FRANCAISE HEMATOLOGIE, SPRINGER INTERNATIONAL, XX, vol. 29, no. 1, 1987, pages 93-96, XP000573465 ISSN: 0029-4810

## Description

The present invention relates to a blood plasma pooled from donors which are substantially of non-Caucasians, a pharmaceutical preparation comprising the blood plasma of the invention and the use of the blood plasma of the invention for the manufacturing of a medicament.

### Background of the invention

Blood groups and the inherent inter-individual differences in human blood were discovered by Karl Landsteiner. The ABO blood group system comprises 4 main phenotypes; 0, A, B, and AB, the phenotype being governed by codominant alleles at the ABO locus on chromosome 9.

Transfusion of ABO-identical or compatible plasma, such as FFP of specific blood groups is an effective and generally well tolerated treatment of various types of complex or isolated coagulation factor deficiencies, in thrombotic thrombocytopenic purpura, and in repeated large volume plasma exchange. However plasma transfusion in principle carries some risk of adverse events among recipients, which include both transmission of infectious and non-infectious diseases.

Non-infectious adverse events typically occur when immunologic incompatibility between e.g. transfused donor red blood cells and recipient antibodies produce accelerated destruction of transfused cells. According to Landsteiner's law, any human individual has antibodies in plasma if the corresponding antigen is absent from the red blood cells. For example, by infusing plasma from a group A donor to a group B patient, anti-B antibodies from donors plasma will react with and lead to destruction of the patient's red blood cells. Similarly, plasma from a group B donor, which contains anti-A antibodies, is incompatible with a blood group A patient; and plasma from a group 0 donor, which contains both anti-A and anti-B antibodies, is incompatible with a patient having blood group A, B, or AB. Therefore, the blood types must be matched to avoid a reaction based on ABO incompatibility.

In addition to non-infectious adverse events, many infectious agents, including viruses, bacteria, and parasites, can be transmitted through blood transfusion. Well recognized viruses include hepatitis A virus (HAV), hepatitis B Virus (HBV), hepatitis C Virus (HCV), human immunodeficiency virus types 1 and 2 (HIV-1/2), and human parvovirus (PV). The risk of transmission of viral infections is minimized by the introduction of donor screening and new test procedures, and in particular, by the introduction of virus inactivation and/or virus removal procedures. Such procedures include virus inactivation by solvent detergent treatment (EP-A-0 131 740), irradiation, and pasteurization, or virus removal by nanofiltration.

Solvent detergent treated human plasma with specific blood groups, such as Octaplas^{®} of blood groups A, B, 0, or AB (Octapharma AG Switzerland), was already developed as an alternative to FFP in order to prevent virus transmission.

Universally applicable plasma in principle can be obtained by using only AB plasma, which contains neither anti-A nor anti-B antibodies (IgM and IgG), thus is compatible with any patient regardless of his blood group. However, the frequency of AB donors (4%) is limited. A plasma suitable for universal transfusion is obtained, if anti-A and/or anti-B antibodies from blood group B and A donors, respectively are removed and/or neutralised by optimal mixing of plasma with the different blood groups. Such neutralization of antibodies was already described (WO-A-99/07390) by mixing 6 to 10 parts of blood or blood plasma of blood group A, 1 to 3 parts of blood or blood plasma of blood group B, and optionally 0 to 1.5 parts of blood or blood plasma of blood group AB without admixing substantial amounts of blood or blood plasma derived from blood group 0.

The CN 1321468 discloses a freeze-dried plasma suitable for various clinical patients with different blood types. It is made by mixing 5-10 portions of plasma type A, 2-7 portions of plasma type B, 0.5-3 portions of plasma type 0 and 0.5-3 portions of plasma type AB.

All human races in principle share the same blood system, although the frequency of the four main ABO blood groups varies in populations throughout the world. Measuring the titres of anti-A and anti-B antibodies, it was surprisingly found that not only the frequency of ABO blood groups but also the titers of blood group specific antibodies differ between different ethnic groups. In the Caucasians, in general, the titers of anti-A in group B and group 0 subjects tend to be higher than the titers of anti-B in group A and group 0 subjects. On the contrary, in people with non-Caucasian background, such as African-American, Hispanic or Native-American donors, anti-B is almost as high as anti-A titers. Consequently, mixing Caucasian plasma with a considerable portion of non-Caucasian origin at the above mentioned ratios, no optimal neutralization of blood group specific antibodies was found. For example, by mixing of 7 parts of blood group A plasma with 3 parts of blood group B plasma, a considerable portion of which was collected from non-Caucasian donors, high anti-B titres, both of IgM and IgG-type, were found in the plasma pool mixture.

### Description of the invention

One object of the invention was to develop a further applicable virus inactivated blood plasma, which is produced by optimal mixing of blood plasma of different blood groups, obtained from blood or plasma of Caucasian origin and portions of non-Caucasian donors, such as donors of African-American, Hispanic and native American origin, facilitating an optimal neutralization of blood group specific antibodies in the mixture.

This object is solved by a blood plasma for human use pooled from donors which belong to 10 % or more to a non-Caucasian population, the plasma obtainable by mixing blood or blood plasma of blood groups A and B, optionally AB without admixing blood or blood plasma of blood group 0 which comprises
- five to six parts of blood or blood plasma from donors having the blood group A,
- four to five parts of blood or blood plasma from donors having the blood group B,
- zero to one part of blood or blood plasma from donors having the blood group AB.

Fractions of blood group 0 can be present in the plasma of the invention so long as these fractions do not introduce antibodies exceeding substantially the overall A or B blood group antigen concentration.

In the blood plasma product of the invention, ABO blood group specific antibodies are essentially neutralized by free blood group substances by an optimal mix of different blood groups, and therefore, this plasma can be transfused regardless of the patient's ABO blood group. Therefore, the blood plasma of the invention further reduces both, the risk of transfusion related infections as well as ABO incompatibility related fatalities.

The ABO blood group specific antibody titre of the blood plasma of the invention is in particular lower than 16 for anti-A and anti-B IgM antibodies, and lower than 64 for anti-A and anti-B IgG antibodies. In another mixture of the blood plasma of the invention, the titre of the anti-A and anti-B IgM antibodies is lower than 8, and the titre of anti-A and anti-B IgG antibodies is lower than 32, employing assays known to a skilled person and described in the European Pharmacopeia (indirect Coombs Test).

Preferably, the blood plasma of the invention is inactivated by the method of EP-A-131740, known as solvent/detergent treatment, irradiation, pasteurisation and/or nanofiltration. A typical solvent/detergent-treatment is for instance use of detergents such as oxyethylated polyphenols, like Triton-X-100, and/or polyoxyethylene derivatives of fatty acids such as Tween 80 and tri-N-butylphosphate (TNBP), or combinations thereof. Also medium to long-chain fatty acids or salts thereof, both saturated and unsaturated, preferably caprylic acid or its salts, can be used for virus inactivation. Other methods are irradiation, pasteurization or nanoflitration. All these methods are known to the person skilled in the art.

Preferably, the blood plasma of the invention is frozen or lyophilized.

The blood plasma of the invention shows coagulation activities comparable to fresh frozen plasma.

The present invention is further illustrated by the following example.

### Example 1

190 kg of fresh frozen plasma of blood group A, 156 kg of plasma of blood group B, and 34 kg plasma of blood group AB, all obtained in a considerable portion from non-Caucasian donors, are mixed after thawing at +37 °C. The obtained plasma mixture is virus inactivated by using the solvent detergent method. After removal of the virus inactivating reagents and freeze-drying, the amount of free anti-A and anti-B antibodies of both IgM and IgG-type is measured. The titre of anti-A and anti-B antibodies of IgM-type is lower than 8 and the titer of anti-A and anti-B antibodies of IgG-type is lower than 32.

### Example 2

205 kg of fresh frozen plasma of blood group A, and 137 kg of plasma of blood group B, all obtained in a considerable portion from non-Caucasian donors, are mixed after thawing at +37 °C . The same procedure as in example 1 was used. The titre of anti-A and anti-B antibodies of IgM-type are lower than 8 and of IgG-type lower than 32.

## Claims

1. A blood plasma for human use pooled from donors which belong to 10 % or more to a non-Caucasian population, the plasma obtainable by mixing blood or blood plasma of blood groups A and B, optionally AB without admixing blood or blood plasma of blood group 0 **characterized in that**
- five to six parts of blood or blood plasma from donors having the blood group A,
- four parts to five parts of blood or blood plasma from donors having the blood group B,
- zero to one part of blood or blood plasma from donors having the blood group AB.

2. The blood plasma according to claim 1 virus-inactivated by any virus inactivation or virus removal method.

3. The blood plasma according to claim 2 wherein the blood plasma was inactivated by solvent/detergent treatment, irradiation, pasteurisation and/or nanofiltration.

4. The blood plasma according to claim 3 wherein the virus inactivation was performed by using detergents such as oxyethylated polyphenols, like Triton-X-100, and/or polyoxyethylene derivatives of fatty acids such as Tween 80 and tri-N-butylphosphate (TNBP), or combinations thereof.

5. The blood plasma according to claim 3 virus inactivated by treatment with long-chain fatty acids, such as caprylic acid or the respective salts.

6. The blood plasma according to any of the forgoing claims substantially free of virus inactivating agents.

7. The blood plasma of any one of the foregoing claims having ABO blood group specific antibody titre lower than 16 for anti-A and anti-B IgM antibodies, and lower than 64 for anti-A and anti-B IgG antibodies.

8. The blood plasma of any of the foregoing claims in liquid, frozen, dried, or lyophilised form.

9. A pharmaceutical composition comprising the blood plasma of any one of the claims 1 to 8.

10. Use of the blood plasma of any of the foregoing claims for the manufacturing of a medicament for the treatment of coagulation factor deficiencies, thrombotic purpura, and in repeated large volume plasma exchange.

11. A process for manufacturing the blood plasma of any one of the claims 1 to 8 by admixing
- four to eight parts of blood or blood plasma from donors having the blood group A,
- more than three parts to seven parts of blood or blood plasma from donors having the blood group B,
- zero to two parts of blood or blood plasma from donors having the blood group AB.

## Patentansprüche

1. Blutplasma zur humanen Verwendung, vereinigt von Spendern, die zu 10% oder mehr zu einer nichteuropäischstämmigen Bevölkerung gehören, wobei das Plasma durch Mischen von Blut oder Blutplasma der Blutgruppen A und B und gegebenenfalls AB erhältlich ist, ohne Blut oder Blutplasma der Blutgruppe 0 zuzumischen, **dadurch gekennzeichnet, dass**
- fünf bis sechs Teile Blut oder Blutplasma von Spendern mit der Blutgruppe A;
- vier bis fünf Teile Blut oder Blutplasma von Spendern mit der Blutgruppe B;
- null bis ein Teil Blut oder Blutplasma von Spendern mit der Blutgruppe AB
gemischt werden.

2. Blutplasma gemäß Anspruch 1, das einer Virusinaktivierung durch irgendein Virusinaktivierungs- oder Virusentfernungsverfahren unterzogen wurde.

3. Blutplasma gemäß Anspruch 2, wobei das Blutplasma durch Lösungsmittel/Tensid-Behandlung, Bestrahlung, Pasteurisierung und/oder Nanofiltration inaktiviert wurde.

4. Blutplasma gemäß Anspruch 3, wobei die Virusinaktivierung unter Verwendung von Tensiden, wie oxyethylierten Polyphenolen, wie Triton X-100, und/oder Polyoxyethylen-Derivaten von Fettsäuren, wie Tween 80, und Tri-n-butylphosphat (TNBP) oder Kombinationen davon durchgeführt wurde.

5. Blutplasma gemäß Anspruch 3, das einer Virusinaktivierung durch Behandlung mit langkettigen Fettsäuren, wie Caprylsäure oder den jeweiligen Salzen, unterzogen wurde.

6. Blutplasma gemäß einem der vorstehenden Ansprüche, das im Wesentlichen frei von Virusinaktivierungsmitteln ist.

7. Blutplasma gemäß einem der vorstehenden Ansprüche, das einen ABOblutgruppenspezifischen Antikörpertiter von weniger als 16 für Anti-A- und Anti-B-IgM-Antikörper und weniger als 64 für Anti-A- und Anti-B-IgG-Antikörper hat.

8. Blutplasma gemäß einem der vorstehenden Ansprüche in flüssiger, gefrorener, getrockneter oder lyophilisierter Form.

9. Pharmazeutische Zusammensetzung, die das Blutplasma gemäß einem der Ansprüche 1 bis 8 umfasst.

10. Verwendung des Blutplasmas gemäß einem der vorstehenden Ansprüche zur Herstellung eines Medikaments zur Behandlung von Koagulationsfaktormängeln, thrombozytopenischer Purpura und bei wiederholtem großvolumigen Plasmaaustausch.

11. Verfahren zur Herstellung des Blutplasmas gemäß einem der Ansprüche 1 bis 8 durch Mischen von:
- vier bis acht Teilen Blut oder Blutplasma von Spendern mit der Blutgruppe A;
- mehr als drei bis sieben Teilen Blut oder Blutplasma von Spendern mit der Blutgruppe B;
- null bis zwei Teilen Blut oder Blutplasma von Spendern mit der Blutgruppe AB.

## Revendications

1. Plasma sanguin pour une utilisation chez l'Homme regroupé à partir de donneurs qui appartiennent à raison de 10% ou plus à une population non caucasienne, le plasma pouvant être obtenu en mélangeant du sang ou du plasma sanguin des groupes sanguins A et B, éventuellement AB, sans mélange de sang ou de plasma sanguin du groupe sanguin O **caractérisé en ce que**
- cinq à six parties de sang ou de plasma sanguin proviennent de donneurs du groupe sanguin A,
- quatre parties à cinq parties de sang ou de plasma sanguin proviennent de donneur du groupe sanguin B,
- zéro à une partie de sang ou de plasma sanguin provient de donneurs du groupe sanguin AB.

2. Plasma sanguin selon la revendication 1 viro-inactivé par tout procédé d'inactivation de virus ou d'élimination de virus.

3. Plasma sanguin selon la revendication 2, dans lequel le plasma sanguin est inactivé au moyen d'un traitement par solvant/détergent, une irradiation, une pasteurisation et/ou une nanofiltration.

4. Plasma sanguin selon la revendication 3, dans lequel l'inactivation de virus est réalisée au moyen de détergents tels que les polyphénols oxyéthylés, tel Triton-X-100, et/ou des dérivés polyoxyéthylène d'acides gras tels que Tween 80 et le tri-N-butylphosphate (TNBP), ou des combinaisons de ceux-ci.

5. Plasma sanguin selon la revendication 3 viro-inactivé au moyen d'un traitement avec des acides gras à longue chaîne tels que l'acide caprylique ou leurs sels respectifs.

6. Plasma sanguin selon l'une quelconque des revendications précédentes essentiellement exempt d'agents inactivateurs de virus.

7. Plasma sanguin selon l'une quelconque des revendications précédentes ayant un titre d'anticorps spécifiques du groupe sanguin ABO inférieur à 16 pour les anticorps anti-IgM A et anti-IgM B, et inférieur à 64 pour les anticorps anti-IgG A et anti-IgG B.

8. Plasma sanguin selon l'une quelconque des revendications précédentes sous forme liquide, congelée, séchée ou lyophilisée.

9. Composition pharmaceutique comprenant le plasma sanguin selon l'une quelconque des revendications 1 à 8.

10. Utilisation du plasma sanguin selon l'une quelconque des revendications précédentes dans la fabrication d'un médicament pour le traitement de déficiences en facteur de coagulation, de purpura thrombotique et pour des échanges plasmatiques en grand volume répétés.

11. Procédé de fabrication de plasma sanguin selon l'une quelconque des revendications 1 à 8 en mélangeant
- quatre à huit parties de sang ou de plasma sanguin provenant de donneurs du groupe sanguin A,
- plus de trois parties à sept parties de sang ou de plasma sanguin provenant de donneur du groupe sanguin B,
- zéro à deux parties de sang ou de plasma sanguin provenant de donneurs du groupe sanguin AB.
